# EUROPÄISCHE PATENTSCHRIFT

(11) **EP 2 491 874 B1**
(45) Veröffentlichungstag und Bekanntmachung des Hinweises auf die Patenterteilung: **25.12.2019**
(21) Anmeldenummer: 11156053.8
(22) Anmeldetag: 25.02.2011
(51) Int. Cl.: A61B 17/29, A61B 18/14, A61B 17/00

(54) **Chirurgisches Instrument mit verbesserter Handhabbarkeit**
Surgical instrument with improved handling
Instrument chirurgical doté d'une maniabilité améliorée

(43) Veröffentlichungstag der Anmeldung: 29.08.2012
(73) Patentinhaber: Erbe Elektromedizin GmbH, 72072 Tübingen (DE)
(72) Erfinder: Amann, Tobias, 72351 Geislingen (DE); Düppuis, Martina, 72124 Pliezhausen (DE); Kühner, Ralf, 70567 Stuttgart (DE); Schmidt Steffanie, 72124 Pliezhausen (DE)
(74) Vertreter: Rüger Abel Patentanwälte PartGmbB

(56) Entgegenhaltungen:
- WO-A1-2010/009525
- DE-U1- 29 804 860
- US-A- 5 984 939
- US-A1- 2010 234 687

## Beschreibung

Die Erfindung betrifft ein chirurgisches Instrument.

Beispielsweise zum Präparieren, Koagulieren, Thermofusionieren und/oder Dissezieren sind chirurgische Instrumente in Gebrauch, die vom Chirurg bei offenchirurgischen oder auch laparoskopischen oder endoskopischen Eingriffen genutzt werden, um gewünschte Eingriffe durchzuführen. Ein solches Instrument ist z.B. aus der DE 101 56 917 A1 bekannt. Dieses Instrument ist für die endoskopische Chirurgie vorgesehen und weist einen langen schaftartigen Werkzeugteil auf, an dessen Ende eine Schere oder Fasszange mit beweglichen Teilen als Werkzeug angeordnet ist. Das proximale Ende des Schafts ist an einem Gehäuse gehalten, das an der Unterseite in einen festen Griffteil übergeht. Dem festen Griffteil ist ein beweglicher Griffteil zugeordnet. Beide Griffteile können wie eine Schere ergriffen und gegeneinander verschwenkt werden, wodurch das am distalen Ende des Schafts gehaltene Werkzeug betätigt wird.

Das Instrument ist für den einhändigen Gebrauch ausgelegt, wobei der Chirurg das Instrument je nach Bedarf in die verschiedensten Richtungen dreht. Dabei kann es dazu kommen, dass er eine unnatürliche Handstellung einnimmt, was zu vorzeitiger Ermüdung, zum Verlust der Feinmotorik oder anderen Unzuträglichkeiten führen kann.

Aus der DE 298 04 860 Ul ist ein Chirurgisches Instrument bekannt, das einen Werkzeugteil und einen entlang einer Längsachse angeordneten Schaft aufweist. An dem distalen Ende desselben ist ein Werkzeug angeordnet, das zwei bewegliche Werkzeugglieder aufweist. Der Schaft ist mit einem Griffteil verbunden, der einen Haltegriff und ein Betätigungsglied aufweist. Das Betätigungsglied ist mit den beweglichen Werkzeuggliedern wirkverbunden. Zwischen dem Schaft und dem Griffteil ist ein mehrachsiges Kugelgelenk vorgesehen. Die von diesem Kugelgelenk festgelegten Achsen liegen außerhalb des Griffteils.

Ein anderes Instrument ist aus der US 5,984,939 bekannt. Es weist einen Handgriff auf, an dem ein Schaft schwenkbar gehalten ist. Die Schwenkachse ist quer zum Griff orientiert.

Davon ausgehend ist es Aufgabe der Erfindung, ein chirurgisches Instrument der genannten Art zu schaffen, bei dem solchen Nachteilen abgeholfen ist.

Diese Aufgabe wird mit dem Instrument nach Anspruch 1 gelöst:
Das erfindungsgemäße Instrument ist in einen Werkzeugteil und einem Griffteil untergliedert. Der Werkzeugteil trägt vorzugsweise einen länglichen und somit eine Längsachse festlegenden Schaft, der an seinem distalen Ende ein chirurgisches Werkzeug trägt. Dieses weist zumindest ein aktivierbares Werkzeugglied auf. Die Aktivierung des Werkzeugglieds kann in einer Bewegung, z.B. Schwenkbewegung desselben liegen, z.B. wenn das Werkzeugglied als Klemme, Zange oder dergleichen dient. Die Aktivierung kann auch in anderen Bewegungen, beispielsweise einer Verschiebebewegung liegen, z.B. wenn das Werkzeugglied als Messer dient. Die Aktivierung kann auch in einer sonstigen Maßnahme, z.B. Erwärmung, Bestromung oder dergleichen oder eine Kombination der vorgenannten Aktivierungen liegen.

Der Griffteil umfasst einen Haltegriff und ein Betätigungsglied. Letzteres steht mit dem aktivierbaren Werkzeugglied in Wirkverbindung. Handelt es sich bei dem aktivierbaren Werkzeugglied um ein bewegliches Werkzeugglied, besteht die Wirkverbindung beispielsweise in einer Getriebeverbindung, so dass das bewegliche Werkzeugglied von dem Betätigungsglied bewegt werden kann.

Der Werkzeugteil und der Griffteil sind durch ein wenigstens zweiachsiges Gelenk miteinander verbunden. Die beiden Gelenkachsen sind dabei quer zu der von dem Schaft festgelegten Längsachse orientiert. Damit kann der Chirurg den Werkzeugteil und den Griffteil so gegeneinander verdrehen, dass er in jedem Bedarfsfall zu einer ergonomischen Handhaltung gelangt. Dadurch wird langfristig ermüdungsfreies und feinfühliges Arbeiten ermöglicht.

Vorzugsweise sind sowohl der Werkzeugteil als auch der Griffteil jeweils als Gehäuse ausgebildet. Getriebemittel und sonstige Komponeneten, wie beispielsweise Schalter, Leiterplatten und dergleichen können in diesem Gehäuse untergebracht sein. Der Werkzeugteil und der Griffteil können beispielsweise aus Kunststoff bestehen. Sie können jeweils ein- oder mehrteilig aufgebaut sein.

Der Griffteil steht vorzugsweise wie ein Pistolengriff von dem Werkzeugteil ab. Vorzugsweise ist die erste Gelenkachse innerhalb des Griffteils angeordnet, so dass sie durch die Faust des Operateurs verläuft. Die zweite Gelenkachse ist vorzugsweise sowohl quer zu dem Griffteil wie auch quer zu der Längsachse des Schafts angeordnet. Die Gelenkachsen und die Längsachse können dabei miteinander spitze Winkel, rechte Winkel oder auch stumpfe Winkel einschließen.

Wenigstens die quer zum Griffteil und quer zum Schaft liegende Gelenkachse weist naturgemäß eine Drehbereichsbegrenzung auf. Bezüglich des Schafts kann der Griffteil zu dem Schaft hin oder von dem Schaft weg geschwenkt werden, wobei der Schwenkwinkel vorzugsweise wenigstens 10°, vorzugsweise mehr, beträgt.

Die quer zum Schaft, dabei aber in dem Griffteil liegende Gelenkachse kann prinzipiell ohne Drehbereichsbegrenzung ausgebildet sein. Der Chirurg kann dann den Griffteil beliebig oft in gleicher Richtung gegen den Werkzeugteil verdrehen. Es kann aber auch zweckmäßig sein, auch hier eine Drehbereichsbegrenzung auf einen Wert von größer als 360° oder auch einen Drehbereich von gleich oder kleiner als 360° vorzusehen. Die Drehbereichsbegrenzung erleichtert die Schaffung von Getriebeverbindungen oder von elektrischen Verbindungen zwischen dem Betätigungsglied des Griffteils und dem Werkzeugteil. Es kann dann sowohl auf berührungslose wie auch auf berührungsaktive Signalübertragungen wie beispielsweise elektrische Schleifringe, Drehtransformatoren, mechanische Drehkupplungen und dergleichen verzichtet werden. Dies ermöglicht beispielsweise eine einfache leitungsgebundene Signalübertragung.

Das wenigstens zweiachsige Gelenk kann im einfachsten Fall als Kugelgelenk ausgebildet sein. Zu ihm gehö ren dann eine Gelenkkugel und eine Gelenkpfanne. Beispielsweise kann die Gelenkkugel den Griffteil und die Gelenkpfanne dem Werkzeugteil zugeordnet sein. Eine umgekehrte Anordnung ist möglich. Das Kugelgelenk legt drei zueinander rechtwinklige Gelenkachsen fest, von denen eine zu der Längsachse parallel ist. Diese kann durch zusätzliche Maßnahmen, bspw. eine bezüglich dieser Achse steife Getriebeverbindung zwischen dem Griffteil und dem Werkzeugteil blockiert werden.

Vorzugsweise stehen die Gelenkkugeln und die Gelenkpfanne reibschlüssig in Eingriff, wodurch eine gewisse Selbsthemmung des Gelenks gegeben ist. Zur Unterstützung der Selbsthemmung kann eine Klemmeinrichtung vorgesehen sein. Die Klemmeinrichtung kann beispielsweise ein ständig wirkendes Klemmglied aufweisen. Z.B. können die Gelenkpfanne und/oder der Gelenkkopf aus federndem Material ausgebildet oder durch Federmittel in Radialrichtung aufeinander zu gespannt sein. Dies erhöht die Reibung zwischen Gelenkpfanne und Gelenkkugel, so dass einmal eingestellte Relativpositionen sicher beibehalten werden. Es können zusätzliche Arretiereinrichtungen, wie Exzenterhebel und dergleichen vorgesehen sein, um die Klemmung bedarfsweise zu verstärken.

Alternativ kann die Selbsthemmung durch eine Rasteinrichtung unterstützt sein. Die Rasteinrichtung kann Vorsprünge und Vertiefungen umfassen. Die Vorsprünge können an der Gelenkkugel oder der Gelenkpfanne angeordnet sein. Die Vertiefungen sind dann an dem jeweils anderen Teil angeordnet. Sind die Vorsprünge und Vertiefungen klein genug und dicht genug beieinander angeordnet, kann die sich ergebende stufenweise Verstellbarkeit den Anforderungen an die Ergonomie dennoch genügen. Die Vertiefungen und/oder die Vorsprünge können in Radialrichtung federnd angeordnet oder ausgebildet sein. Beispielsweise können sie mit der Gelenckugel oder der Gelenkpfanne einstückig ausgebildet sein. In der Gelenkkugel oder der Gelenkpfanne vorgesehene Schlitze können zwischen einander federnde Zungen festlegen, die die Vorsprünge und/oder Vertiefungen tragen und somit in Radialrichtung federnde Rastglieder bilden.

Die Gelenkkugel kann einen Durchgangskanal aufweisen, der von dem Innenraum des Werkzeugteils in den Innenraum des Griffteils führt. Dieser Durchgangskanal kann eine oder mehrere Kraftübertragungsglieder aufnehmen, die die Bewegung des Betätigungsglieds auf das aktivierbare Werkzeugglied übertragen. Das Getriebe kann ein Hebelgestänge oder ein Zugmittelgetriebe sein. Das Hebelgetriebe kann aus ein oder mehreren miteinander gelenkig verbundenen Hebeln bestehen. Zur gelenkigen Verbindung können Filmscharniere dienen.

Bei einer vorteilhaften Ausführungsform ist zur Umsetzung der Betätigungsbewegung des Betätigungsglieds am Griffteil in eine lineare Betätigungsbewegung im Werkzeugteil lediglich ein einziges Getriebeglied vorgesehen. Dies ergibt einen einfachen robusten Aufbau. Das Getriebeglied kann ein mehrgliedriger Hebel mit flexiblem Abschnitt, z.B. Filmscharnier, oder auch ein flexibles Zugmittel sein, das frei gespannt ist oder über eine oder mehrere Umlenkstellen läuft, oder ein starrer Hebel, der nur mit einer Stirnseite auf einer von dem Betätigungsglied bewegten Druckfläche steht.

Um die Drehbewegung des Griffteils in Bezug auf das Werkzeugteil wie oben beschrieben zu ermöglichen, wird bei der Verwendung eines Getriebegliedes beispielsweise eine Kugellagerung vorgeschlagen. Diese Kugellagerung kann sowohl im Griffteil oder alternativ im Werkzeugteil angeordnet sein, wobei die Gelenkkugel am Getriebeglied und die Gelenkpfanne am Werkzeugteil oder am Griffteil angeordnet sein kann. Alternativ kann die Gelenkpfanne auch am Getriebeteil und die Gelenkkugel am Werkzeugteil oder am Griffteil angeordnet sein.

Weitere Einzelheiten vorteilhafter Ausführungsformen ergeben sich aus der Zeichnung, Unteransprüchen oder der Beschreibung. Es zeigen:
Fig. 1 ein chirurgisches Instrument in perspektivischer schematisierter Darstellung,
Fig. 2 das chirurgische Instrument nach Fig. 1, mit vom Werkzeugteil getrenntem Griffteil in schematisierter Perspektivdarstellung,
Fig. 3 und 4 das chirurgische Instrument in einer ersten Ausführungsform in ausschnittsweiser Schnittdarstellung, mit Griffteil in Mittelstellung in zwei verschiedenen Betätigungsstellungen,
Fig. 5 und 6 das chirurgische Instrument nach Fig. 3, mit nach vorn geschwenktem Griffteil in zwei verschiedenen Betätigungsstellungen,
Fig. 7 und 8 das chirurgische Instrument nach Fig. 3 und 4, mit nach hinten geschwenktem Griffteil in zwei verschiedenen Betätigungsstellungen,
Fig. 9 und 10 eine alternative Ausführungsform des chirurgischen Instruments in schematisierter ausschnittsweiser Schnittdarstellung in zwei verschiedenen Betätigungsstellungen,
Fig. 11 und 12 eine dritte Ausführungsform des chirurgischen Instruments in ausschnittsweiser geschnittener Darstellung in zwei verschiedenen Betätigungsstellungen,
Fig. 13 und 14 eine vierte Ausführungsform des chirurgischen Instruments in ausschnittsweiser geschnittener Darstellung in zwei verschiedenen Betätigungsstellungen
Fig. 15 ein Kugelgelenk mit verbesserter Klemmung, in schematisierter Darstellung,
Fig. 16 ein Kugelgelenk mit Rastmitteln in teilweise geschnittener Explosionsdarstellung.

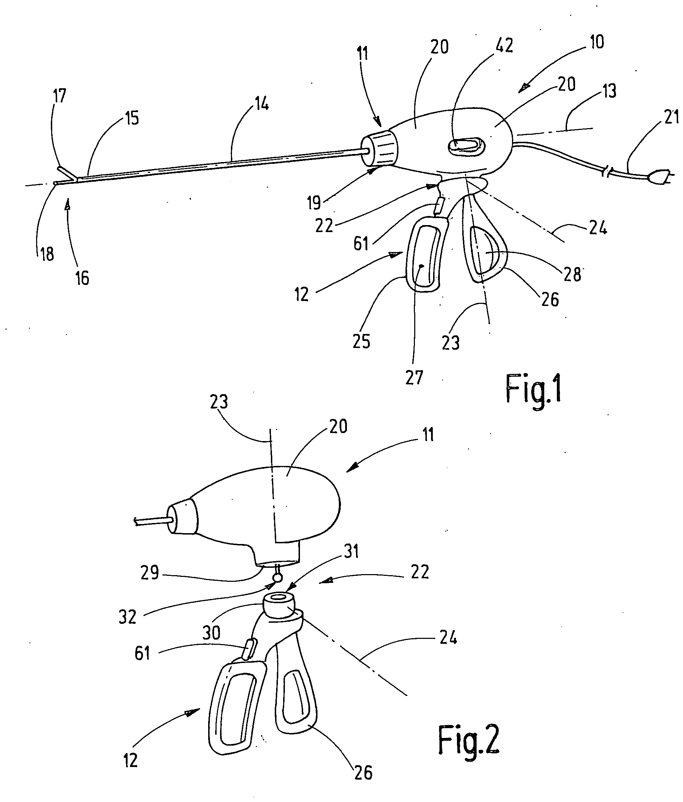

In Fig. 1 ist ein chirurgisches Instrument 10 veranschaulicht, das sowohl für die offene wie auch für die endoskopische Chirurgie geeignet ist. Es handelt sich hier beispielshalber um ein multifunktionales Instrument zur Präparation, zum Fassen, zum Thermofusionieren, zur Dissektion und/oder Koagulation von Geweben, insbesondere Gefäßen.

Das Instrument 10 weist ein Werkzeugteil 11 und einen Griffteil 12 auf. Der Werkzeugteil 11 umfasst einen entlang einer Längsachse 13 angeordneten länglichen, vorzugsweise rohrartigen Schaft 14, der an seinem distalen Ende 15 ein Werkzeug 16 trägt. Dieses weist, zumindest im vorliegenden Ausführungsbeispiel, wenigstens ein bewegliches Werkzeugglied 17 und ein weiteres Werkzeugglied 18 auf, das ebenfalls beweglich oder, wie dargestellt, in Bezug auf den Schaft 14 fest angeordnet sein kann. Der Schaft 14 kann mit dem übrigen Werkzeugteil 11 fest oder, wie dargestellt, über eine Drehkupplung 19 gegebenenfalls auswechselbar verbunden sein. Durch die Drehung des Schafts 14 kann die aus den Werkzeuggliedern 17, 18 gebildete Zange oder Schere um die Längsachse 13 gedreht werden.

Zu dem Werkzeugteil 11 gehört weiter ein Gehäuse 20. Von diesem kann sich eine z.B. mit einem Stecker versehene elektrische Leitung 21 weg erstrecken, die an ein Gerät für den Betrieb des chirurgischen Instruments 10 anschließbar ist. Die Leitung kann allerdings auch an dem Griffteil 12 angeordnet sein.

Das Gehäuse 20 ist mit dem Griffteil 12 über ein wenigstens zweiachsiges Gelenk 22 verbunden. Dieses legt eine erste Gelenkachse 23 fest, die innerhalb des Griffteils 12 liegt. Die Gelenkachse 23 ist quer zu der Längsachse 13 orientiert und schließt mit dieser einen rechten Winkel ein.

Das Gelenk 22 legt eine zweite Gelenkachse 24 fest, die sowohl quer zu der ersten Gelenkachse 23, wie auch quer zu der Längsachse 13 orientiert ist. Der Winkel zwischen den Gelenkachsen 23, 24 kann ein rechter Winkel, ein spitzer Winkel und ein stumpfer Winkel sein. Gleiches gilt für den Winkel zwischen der Gelenkachse 24 und der Längsachse 13.

Der Griffteil 12 umfasst einen fest an dem Griffteil 12 angeordneten Haltegriff 25 und ein gegen den Haltegriff 25 schwenkbares Betätigungsglied 26. Der Haltegriff 25 kann eine große Grifföffnung 27 aufweisen, durch die (alle) Finger einer Hand passen. Das Betätigungsglied 26 ist vorzugsweise mit einer Öffnung 28 versehen, durch die der Daumen des Operateurs passt. Die Anordnung kann jedoch, sowohl was die Beweglichkeit wie auch die Öffnungen der Griffteile betrifft, auch umgekehrt getroffen sein.

Fig. 2 veranschaulicht nochmals gesondert in voneinander getrenntem Zustand den Werkzeugteil 11 und den Griffteil 12 sowie das dazwischen angeordnete Gelenk 22. Dem Gehäuse 20 ist eine Gelenkpfanne 29 zugeordnet. Dem Griffteil 12 ist eine Gelenkkugel 30 zugeordnet. Beide passen leicht klemmend ineinander, so dass sie sich mit moderaten Handkräften gegeneinander verdrehen lassen und ihre einmal eingenommene Drehstellung beibehalten. Die Gelenkkugel 30 kann in der Gelenkpfanne 29 sowohl um die erste Achse 23, wie auch um die zweite Achse 24 gedreht werden. Die Gelenkkugel 30 enthält ein z.B. zylindrischen Durchgangskanal 31 zur Aufnahme wenigstens eines Kraftübertragungsglieds 32, das beispielsweise dazu dient, die Bewegung des Betätigungsglieds 26 über ein Getriebe auf das Werkzeugglied 17 zu übertragen.

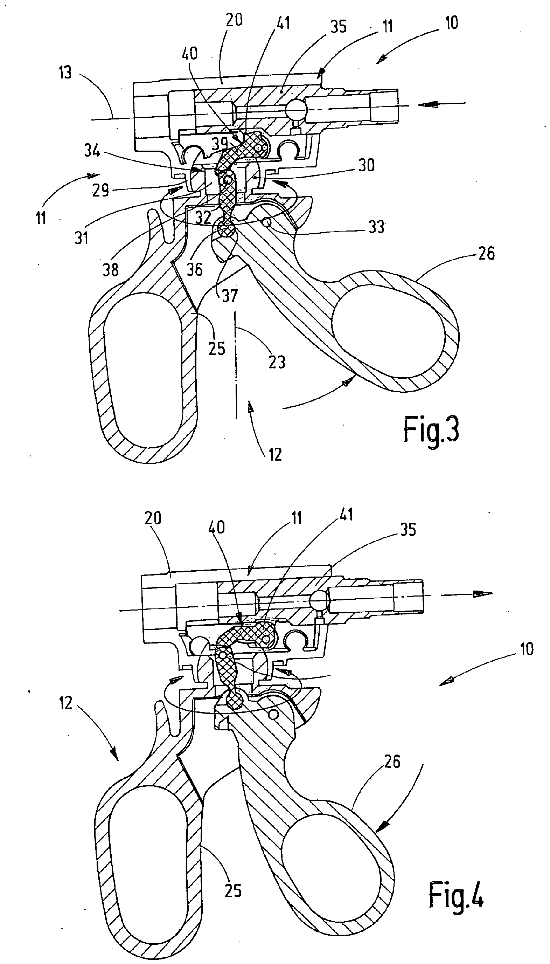

Fig. 3 veranschaulicht das Instrument 10 im Vertikalschnitt und offenbart somit den inneren Aufbau des Werkzeugteils 11 und des Griffteils 12. Wie ersichtlich, ist das Betätigungsglied 26 an dem Haltegriff 25 z.B. mittels eines Zapfens 33 schwenkbar gelagert. Die Schwenkachse kann ungefähr parallel zu der zweiten Gelenkachse 24 angeordnet sein.

Die Schwenkbewegung des Betätigungsglieds 26 wird über ein Getriebe 34 in eine Translationsbewegung eines Schiebers 35 umgesetzt, der in dem Gehäuse 20 verschiebbar gelagert ist und über ein nicht weiter veranschaulichtes Gestänge das Werkzeug 16 betätigt. Das Kraftübertragungsglied 32 gehört zu diesem Getriebe 34. Es ragt durch den Durchgangskanal 31 und weist an seinem unteren Ende eine Gelenckugel 36 auf. Diese sitzt in einer entsprechenden Gelenkpfanne 37, die in einem kurzen Abstand zu dem Zapfen 33 an dem Betätigungsglied 26 ausgebildet ist. Die Kugel 36 ist in der Gelenkpfanne 37 um die erste Gelenkachse 23 oder eine zu dieser parallelen Achse drehbar. Vorzugsweise liegt der Mittelpunkt der Kugel 36 auf der Gelenkachse 23 oder in unmittelbarer Nähe derselben.

Das Kraftübertragungsglied 32 ist ein vorzugsweise gerader Zugstift, der bei einem Scharniergelenk 38 mit einem Arm 39 eines Winkelhebels 40 verbunden ist. Dieser ist in dem Gehäuse 20 des Werkzeugteils 11 schwenkbar gelagert. Sein anderer Arm 41 ist gegabelt und steht mit einer Nut des Schiebers 35 in Eingriff. Eine Schwenkbewegung des Winkelhebels 40 schiebt den Schieber 35 entlang der Längsachse 13 zu dem Werkzeug 16 hin oder von diesem weg.

Das Scharniergelenk 38 zwischen dem Kraftübertragungsglied 32 und dem Arm 39 verhindert ein Verschwenken des Werkzeugteils 11 gegen das Griffteil 12 um eine zu der Achse 13 parallele Achse. Es können weitere Mittel vorgesehen sein, um eine solche (dritte) Schwenkachse zu blockieren, falls sie unerwünscht ist. Sollte diese dritte Schwenkmöglichkeit jedoch gerade gewünscht sein, kann anstelle des Scharniergelenks 38 ein Kugelgelenk oder ein in zwei Richtungen flexibles Zwischenstück als Gelenk vorgesehen sein.

Das Instrument 10 kann weitere Komponenten enthalten, die hier nicht beschrieben sind, so z.B. einen oder mehrere elektrische Schalter 61 (Figur 2) zum Aktivieren eines Werkzeuges 16 beispielsweise einer Elektrode, oder Sperren 42 (Fig. 1), die ein Verschieben des Schiebers 35 und somit einer Betätigung des Werkzeugs 16, oder dergleichen Mittel ganz oder teilweise verhindern.

Das insoweit beschriebene chirurgische Instrument 10 arbeitet wie folgt:
Zur Benutzung ergreift der Chirurg das Instrument 10 und dreht den Griffteil 12 in die gewünschte Position. Er kann dabei, wie in Fig. 3 veranschaulicht ist, den Werkzeugteil 11 und den Griffteil 12 um die Drehachse 23 gegeneinander verdrehen oder verschwenken. In Fig. 3 ist dies durch einen Rundpfeil angedeutet. Wird der Werkzeugteil 11 gegen den Griffteil 12 verdreht, dreht sich die Kugel 36 in der Gelenkpfanne 37, ohne dass dadurch eine Verschiebung des Schiebers 35 auftritt. Der Chirurg kann auf diese Weise den Schaft 14 in verschiedenste Richtungen drehen, beispielsweise längs zu seinem Arm, quer zu seinem Arm usw., ohne dazu sein Handgelenk unnatürlich bewegen zu müssen.

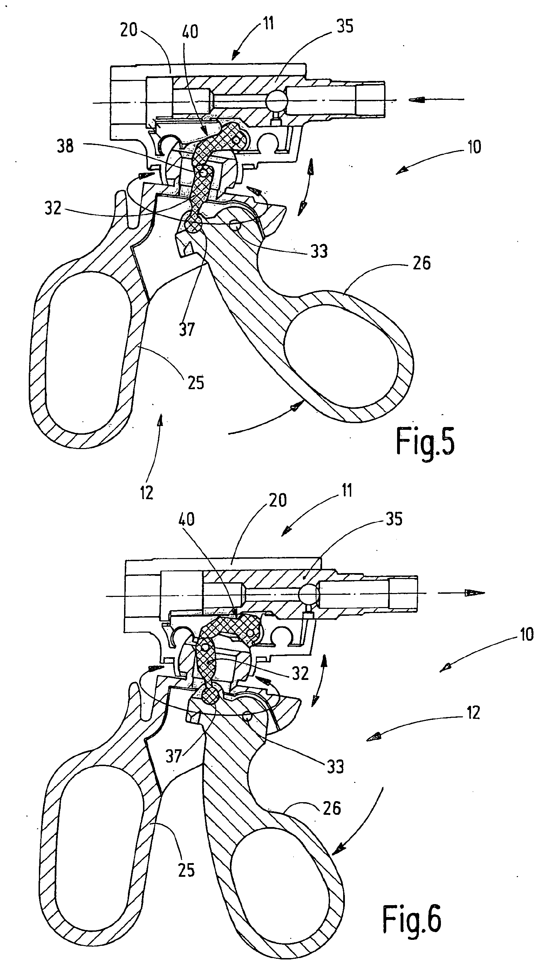

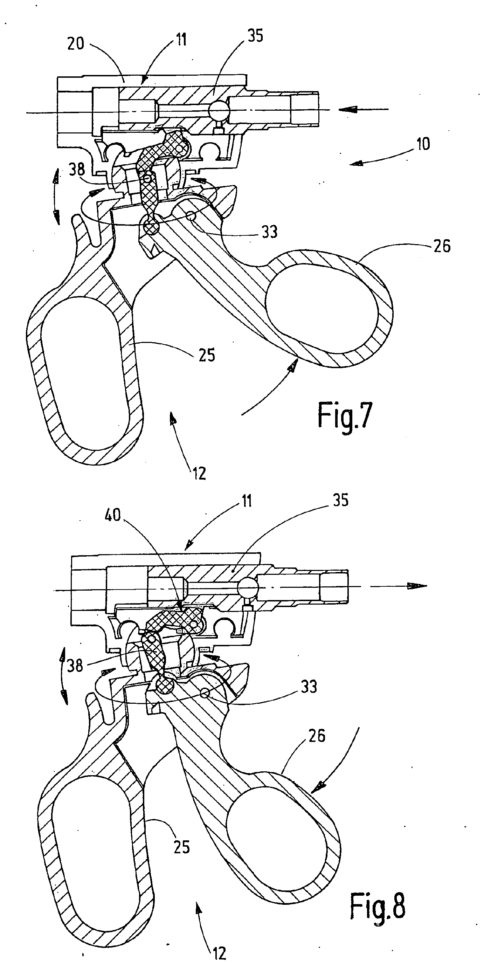

Weiter kann der Chirurg den Werkzeugteil 11 um die zweite Gelenkachse 24 gegen den Griffteil 12 drehen. Die Gelenkachse 24 steht auf den Figuren 3 bis 8 senkrecht. Er kann den Griffteil 12 nach vorn zu dem Schaft 14 hin schwenken, wie es Fig. 5 zeigt, oder nach hinten von dem Schaft 14 weg, wie es Fig. 7 zeigt. Diese Schwenkbewegung hat vorzugsweise keine Verschiebung des Schiebers 35 zur Folge. Dies insbesondere dann nicht, wenn das Gelenk 38, wie es bevorzugt wird, bei nicht betätigtem Werkzeug 16 etwa in der Mitte der Gelenkkugel 30 angeordnet ist. Dies ist in den Figuren 3, 5 und 7 veranschaulicht, die den nicht betätigten Zustand bei verschiedenen Schwenkwinkeln des Griffteils 12 zeigen.

In jeder der Schwenkstellungen des Griffteils 12 in Bezug auf das Werkzeugteil 11 kann das Werkzeug 16 kontrolliert und gezielt bewegt werden, indem das Betätigungsglied 26 geschwenkt wird. Fig. 4 veranschaulicht dies in Bezug auf die rechtwinklig unverschwenkte Stellung des Griffteils 12 bezüglich des Werkzeugteils 11. Werden der Haltegriff 25 und das Betätigungsglied 26, wie in Fig. 4 durch einen bogenförmigen Pfeil angedeutet, z.B. durch Handbewegung aufeinander zu bewegt, wird dadurch das Kraftübertragungsglied 32 nach oben gedrückt. Der Winkelhebel 40 dreht im Uhrzeigersinn und verschwenkt deshalb mit seinem Arm 41 den Schieber 35 nach rechts. Eine nicht weiter veranschaulichte Zugstange betätigt dabei das Werkzeug 16.

In gleicher Weise kann das Werkzeug 16 betätigt werden, wenn der Griffteil 12 gemäß Fig. 5 um die zweite Gelenkachse 24 z.B. zu dem Schaft 14 beziehungsweise dem Werkzeug 16 hin geschwenkt ist. Wird das Betätigungsglied 26 zu dem Haltegriff 25 hin gedrückt und schwenkt es dabei um den Zapfen 33, hebt es mit seiner Gelenkpfanne 37 wiederum das Kraftübertragungsglied 32 an und verschwenkt damit den Winkelhebel 40. Dies hat eine Verschiebung des Schiebers 35 nach rechts zur Folge.

In gleicher Weise arbeitet das Instrument 10, wenn der Griffteil 12 gemäß Fig. 7 um die Gelenkachse 24 von dem Schaft 14 bzw. dem Werkzeug 16 weg geschwenkt ist. In unbetätigtem Zustand, wenn der Haltegriff 25 und das Betätigungsglied 26, wie in Fig. 7 dargestellt, in maximalem Abstand stehen, befindet sich der Schieber 35 in seiner werkzeugnahen Position, d.h. in der Darstellung gemäß Fig. 3 bis 8 links. Wird das Betätigungsglied 26 gemäß Fig. 8 an den Haltegriff 25 herangeschwenkt, wird wiederum das Kraftübertragungsglied 38 nach oben zu dem Werkzeugteil 11 hin gedrückt, wodurch der Winkelhebel 40 schwenkt und der Schieber 35 in Betätigungsrichtung, d.h. in Fig. 8 nach rechts verschoben wird.

Die vorgestellte Kugelgelenkkupplung zwischen Werkzeugteil 11 und Griffteil 12 und das sich durch das Gelenk 22 hindurch erstreckende Getriebe 34, ermöglichen einerseits eine nahezu beliebige Einstellung des Winkels zwischen dem Griffteil 12 und dem Werkzeugteil 11 und andererseits in jeder eingestellten Winkelposition eine uneingeschränkte Betätigung des Werkzeugs 16.

An dem insoweit beschriebenen Instrument 10 sind zahlreiche Abwandlungen möglich. Im Nachfolgenden werden exemplarisch, insbesondere das Getriebe 34 betreffende Abwandlungen betrieben. Diese nachfolgend beschriebenen Abwandlungen des Getriebes 34, wie auch weitere aus der nachfolgenden Beschreibung hervorgehende Details, sind bei der vorstehend beschriebenen Ausführungsform ebenfalls anwendbar. Die nachfolgende Beschreibung beschränkt sich auf Abweichungen der Ausführungsform. Im Übrigen wird unter Zugrundelegung der bereits eingeführten Bezugszeichen auf die vorige Beschreibung verwiesen.

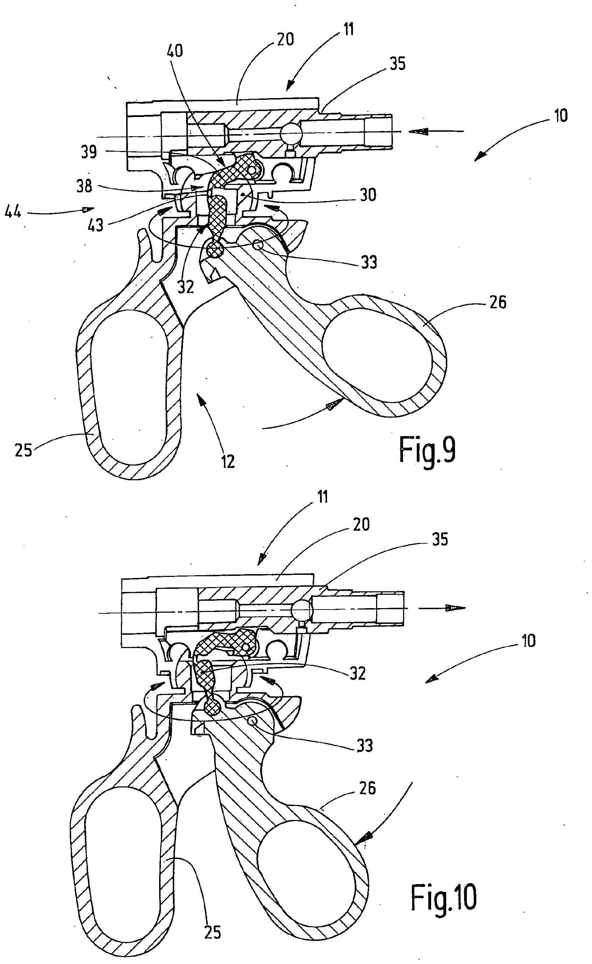

Bei der Ausführungsform nach den Figuren 9 und 10 ist das Scharniergelenk 38 durch einen flexiblen Abschnitt 43 gebildet, über den das stößelartige Kraftübertragungsglied 32 nahtlos einstückig mit dem Arm 39 des Winkelhebels 40 verbunden ist. Mit anderen Worten, der Winkelhebel 40 und das Kraftübertragungsglied 32 sind ein einstückiges Teil vorzugsweise auf Kunststoff mit einem kurzen dünnwandigen Abschnitt 43, der z.B. ein einachsiges Filmscharnier 44 bildet. Das Filmscharnier 44 ist wiederum vorzugsweise in dem Zentrum der Gelenkkugel 30 angeordnet. Die Betätigung erfolgt in allen Dreh- und Winkelstellungen des Griffteils 12 durch Heranschwenken des Bedienteils bzw. Betätigungsglied 26 an den Haltegriff 25, wie in Fig. 10 veranschaulicht. Es wird dabei, wie schon zuvor beschrieben, das Kraftübertragungsglied 32 angehoben, wodurch der Schieber 35 aus der in Fig. 9 veranschaulichten linken Position in die in Fig. 10 veranschaulichte rechte Position überführt wird. Der Abschnitt 34 kann auch in zwei Achsen flexibel ausgebildet sein, um die volle Drehbeweglichkeit des Gelenks 22 um drei Achsen nicht zu stören.

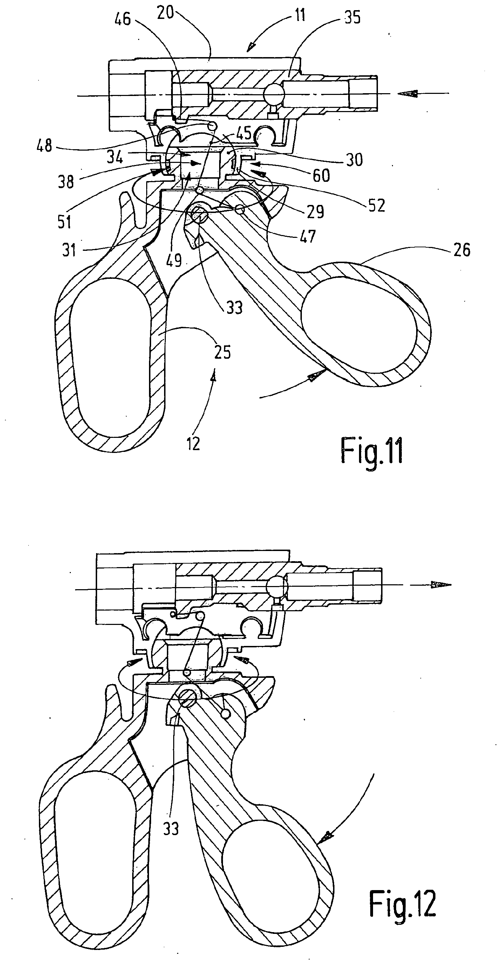

Eine weitere Abwandlung des Getriebes 34 veranschaulichen die Figuren 11 und 12. Als Kraftübertragungsglied 32 ist hier ein Zugmittel 45 z.B. in Gestalt eines dünnen flexiblen Kunststoffdrahts, -bands oder -seils, eines dünnen Stahldrahts oder dergleichen vorgesehen. Das Zugmittel 45 ist mit einem Ende 46 an dem Schieber 35 und mit einem anderen Ende 47 an dem Betätigungsglied 26 befestigt. Das Zugmittel 45 erstreckt sich durch den Durchgangskanal 31 der Gelenkkugel 30, wobei das Zugmittel 45 über eine oder mehrere Umlenkstellen 48, 49 laufen kann, die beispielsweise durch Zapfen gebildet werden. Das Zugmittel 45 kann zwischen den beiden Umlenkstellen 48, 49 mit einer Drehkupplung versehen sein, um ein ungehindertes Verdrehen des Griffteils 12 gegen das Werkzeugteil 11 zu ermöglichen. Alternativ kann die Verdrehungsmöglichkeit um die erste Achse 23 eingeschränkt sein. In Fig. 11 ist dazu ein Drehbegrenzungsmittel 50 veranschaulicht. Als solches kann die Gelenkkugel 30 mit einer in Umfangsrichtung verlaufenden Nut 51 versehen sein, in die ein Stift 52 ragt, der z.B. in die Gelenkpfanne 29 eingesetzt sein kann. Die ringsumlaufende Nut ist an einer Stelle durch eine Wand unterbrochen, die an dem Stift 51 anstößt und so eine zu weite Verdrehung verhindert. Auf diese Weise können Drehwinkel von < 360° festgelegt werden. Außerdem erbringt die Breite der Nut eine Schwenkwinkelbegrenzung für das Verschwenken um die zweite Gelenkachse 24.

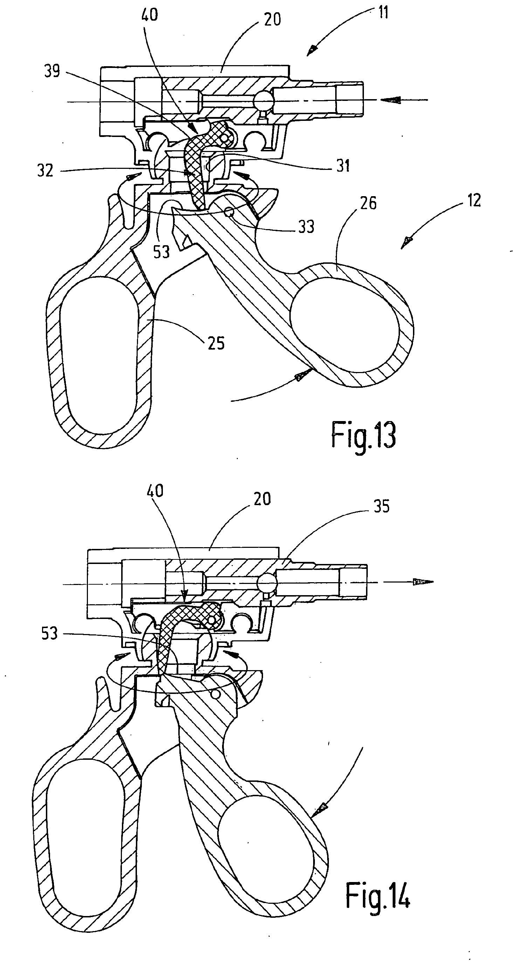

Eine weitere Abwandlung geht aus den Figuren 13 und 14 hervor. Wie schon bei der vorigen Ausführungsform wird davon ausgegangen, dass der Schieber 35 in Richtung auf das Werkzeug 16 hin z.B. durch eine nicht weiter veranschaulichte Feder vorgespannt ist. Bei dieser Ausführungsform ist das Kraftübertragungsglied 32 starrer Bestandteil des Winkelhebels 40 und erstreckt sich als nach unten abgewinkelter Abschnitt des Arms 39 durch den Durchgangskanal 31 hindurch zu einer sphärischen Druckfläche 53, die an einem von dem Zapfen 33 weg ragenden Teil des Betätigungsglieds 26 an der aus dem Durchgangskanal 31 hinreichenden Seite angebracht ist. Auf dieser Druckfläche 53 steht das Kraftübertragungsglied 32 mit seiner gerundeten Stirnfläche. Durch das Verschwenken des Betätigungsglieds 26 gegen den ,Handgriff bzw. Haltegriff 25 wird die Druckfläche 53, wie Fig. 14 zeigt, nach oben bewegt, wodurch der Winkelhebel 40 schwenkt, um den Schieber 35 von links (Fig. 13) nach rechts (Fig. 14) bewegen.

Auch bei dieser Ausführungsform ist eine volle Dreh- und Schwenkmöglichkeit des Griffteils 12 gegen das Werkzeugteil 11 um die beiden Gelenkachsen 23, 24 gegeben. In allen Schwenk- und Drehstellungen kann das Werkzeug 16 ungehindert aktiviert, d.h. betätigt werden.

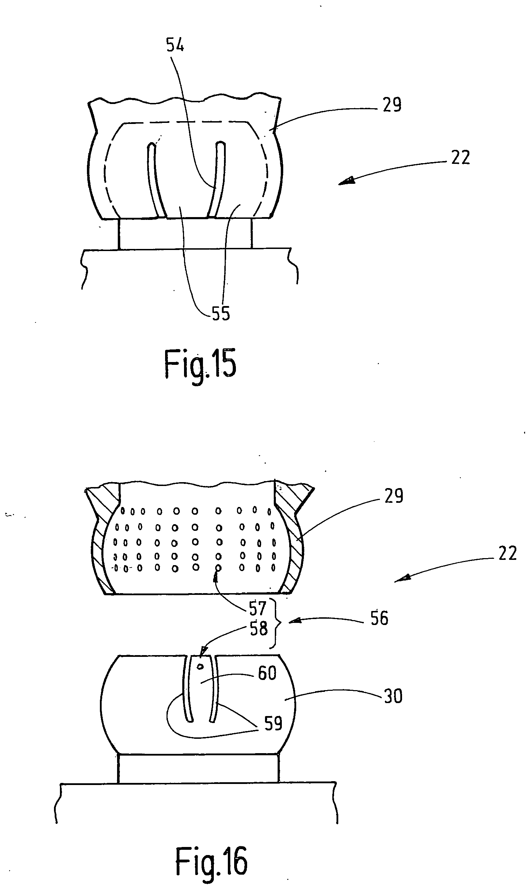

Das Gelenk 22 des Instruments 10 kann eine reibschlüssige Klemmung aufweisen. Eine Ausführungsmöglichkeit dazu ist in Figur 15 veranschaulicht. Die Gelenkpfanne 29 kann in mehrere durch Schlitze 54 voneinander getrennte federnde Finger 55 unterteilt sein, die radial nach innen gegen die Gelenkkugel 30 gespannt sind. Die umgekehrte Anordnung mit einer geschlitzten Gelenkkugel und einer ungeschlitzten Gelenkpfanne ist ebenfalls möglich.

Weiter kann das Gelenk 22 mit einer Rasteinrichtung 56 versehen sein, wie es Figur 16 veranschaulicht. Zu der Rasteinrichtung 56 können Vertiefungen 57 und Vorsprünge 58 gehören. Die Vertiefungen 57 können in die Innenfläche der Gelenkpfanne 29 in einer vorgegebenen Anordnung eingebracht sein. Diese Anordnung kann mehrere Reihen und Spalten von Vertiefungen 57 enthalten, die gewünschte Rastpositionen festlegen. Der zumindest eine Vorsprung 58 kann an einer durch Schlitze 59 freigestellten Zunge 60 der Gelenkkugel 30 angeordnet sein. Bei entsprechender Positionierung von Gelenkkugel 30 und Gelenkpfanne 29 schnappt der Vorsprung 58 in eine Vertiefung 57 und sichert somit rastend die eingenommene Drehposition. Es wird darauf hingewiesen, dass die Zunge alternativ an der Gelenkpfanne und die Vertiefungen an der Gelenkkugel 30 angebracht sein können.

Das erfindungsgemäße chirurgische Instrument 10 weist einen Werkzeugteil 11 und einen Griffteil 12 auf, die über ein Kugelscharnier oder ein sonstiges zweiachsiges Gelenk 22 miteinander verbunden sind. Das Gelenk 22 ermöglicht dem Chirurgen ein ermüdungsfreies, ergonomisches und feinfühliges Arbeiten. Das an dem Werkzeugteil 11 gehaltene Werkzeug 16 ist durch das Betätigungsglied 26 aktivierbar, beispielsweise mechanisch betätigbar. Weitere Betätigungselemente, wie beispielsweise elektrische Schalter oder dergleichen, können an dem Griffteil 12 angeordnet sein, um das Werkzeug 16 zu aktivieren, beispielsweise unter Strom zu setzen. Die Getriebeverbindung zwischen dem Griffteil 12 und dem Werkzeug 16 kann über starre Hebel und Verbindungsstücke oder auch über flexible Elemente, beispielsweise ein Zugseil oder starre Teile mit Filmscharnier erreicht werden. Um eine Schwenkbewegung des Griffteils 12 gegen das Werkzeugteil 11 von einer Betätigung des Werkzeugs 16 zu entkoppeln, ist mindestens ein Gelenk 38 des Getriebes 34 im Zentrum des Gelenks 22 angeordnet.

### Bezugszeichenliste:

- 10: Instrument
- 11: Werkzeugteil
- 12: Griffteil
- 13: Längsachse
- 14: Schaft
- 15: Distales Ende des Schafts 14
- 16: Werkzeug
- 17: Bewegliches Werkzeugglied
- 18: Weiteres Werkzeugglied
- 19: Drehkupplung
- 20: Gehäuse
- 21: Elektrische Leitung
- 22: Gelenk
- 23: Erste Gelenkachse
- 24: Zweite Gelenkachse
- 25: Haltegriff
- 26: Betätigungsglied
- 27: Öffnung
- 28: Öffnung
- 29: Gelenkpfanne
- 30: Gelenkkugel
- 31: Durchgangskanal
- 32: Kraftübertragungsglied
- 33: Zapfen
- 34: Getriebe
- 35: Schieber
- 36: Kugel
- 37: Gelenkpfanne
- 38: Scharniergelenk
- 39: Arm
- 40: Winkelhebel
- 41: Arm
- 42: Sperre
- 43: Flexibler Abschnitt
- 44: Filmscharnier
- 45: Zugmittel
- 46: Erstes Ende des Zugmittels
- 47: Zweites Ende des Zugmittels
- 48: Erste Umlenkstelle
- 49: Zweite Umlenkstelle
- 50: Drehbegrenzungsmittel
- 51: Nut
- 52: Stift
- 53: Druckfläche
- 54: Schlitze
- 55: Finger
- 56: Rasteinrichtung
- 57: Vertiefungen
- 58: Vorsprung
- 59: Schlitze
- 60: Zunge
- 61: Schalter

## Patentansprüche

1. Chirurgisches Instrument (10)
mit einem Werkzeugteil (11), der einen entlang einer Längsachse (13) angeordneten Schaft (14) mit einem an seinem distalen Ende (15) angeordneten Werkzeug (16) aufweist, das wenigstens ein aktivierbares Werkzeugglied (17) aufweist,
mit einem Griffteil (12), der einen Haltegriff (25) und ein Betätigungsglied (26) aufweist, das mit dem aktivierbaren Werkzeugglied (17) in Wirkverbindung steht, mit einem wenigstens zweiachsigen Gelenk (22), über das der Griffteil (12) und der Werkzeugteil (11) untereinander verbunden sind und das eine erste Gelenkachse (23) und eine zweite Gelenkachse (24) aufweist, wobei die erste Gelenkachse (23) in einem rechten Winkel zu der Längsachse (13) und die zweite Gelenkachse (24) quer zu dem Griffteil (12) angeordnet ist
**dadurch gekennzeichnet,**
**dass** die erste Gelenkachse (23) in dem Griffteil (12) liegend angeordnet ist.

2. Instrument nach Anspruch 1, **dadurch gekennzeichnet, dass** das aktivierbare Werkzeugglied (17) beweglich angeordnet und über ein Getriebe (34) mit dem Betätigungsglied (26) verbunden ist.

3. Instrument nach Anspruch 1 oder 2, **dadurch gekennzeichnet, dass** sowohl die erste als auch die zweite Gelenkachse (23, 24) jeweils eine Drehbereichsbegrenzung (50) aufweisen.

4. Instrument nach Anspruch 1, **dadurch gekennzeichnet, dass** das Gelenk (22) ein Kugelgelenk mit einer Gelenckugel (30) und Gelenkpfanne (29) ist.

5. Instrument nach Anspruch 1, **dadurch gekennzeichnet, dass** das Gelenk (22) eine Rasteinrichtung (56) zur Fixierung eingestellter Relativpositionen von Griffteil (12) und Werkzeugteil (11) aufweist.

6. Instrument nach Anspruch 4 und 5, **dadurch gekennzeichnet, dass** die Rasteinrichtung (56) durch zumindest einen Vorsprung (58) und Vertiefungen (57) gebildet ist, die an der Gelenkkugel (30) und der Gelenkpfanne (29) angeordnet sind.

7. Instrument nach Anspruch 4, **dadurch gekennzeichnet, dass** die Gelenkkugel (30) mit dem Griffteil (12) verbunden ist und dass die Gelenkpfanne (29) mit dem Werkzeugteil (11) verbunden ist.

8. Instrument nach Anspruch 4, **dadurch gekennzeichnet, dass** die Gelenkkugel (30) einen Durchgangskanal (31) aufweist, der einen Innenraum des Werkzeugteils (11) mit einem Innenraum des Griffteils (12) verbindet.

9. Instrument nach Anspruch 2 und 4, **dadurch gekennzeichnet, dass** in dem Durchgangskanal (31) ein Kraftübertragungsglied (32) angeordnet ist, das zu dem Getriebe (34) gehört.

10. Instrument nach Anspruch 2, **dadurch gekennzeichnet, dass** das Getriebe (34) ein Hebelgestänge ist.

11. Instrument nach Anspruch 9, **dadurch gekennzeichnet, dass** Kraftübertragungsglied (32) einen flexiblen, als Gelenk (38) dienenden Abschnitt (43) aufweist.

12. Instrument nach Anspruch 11, **dadurch gekennzeichnet, dass** der Abschnitt (43) ein Filmscharnier (44) ist.

13. Instrument nach Anspruch 9, **dadurch gekennzeichnet, dass** das Kraftübertragungsglied (32) ein flexibles Zugmittel (45) ist.

14. Instrument nach Anspruch 1, **dadurch gekennzeichnet, dass** das Griffteil (12) einen Schalter (61) umfasst, um eine Aktivierung des Werkzeuges (16) zu ermöglichen.

## Claims

1. Surgical instrument (10)
with a tool part (11) having a shaft (14) which is arranged along a longitudinal axis (13) and comprises a tool (16) that is arranged at its distal end (15) and comprises at least one activatable tool element (17),
with a handle part (12) having a handle (25) and an actuating element (26) which is actively connected to the activatable tool element (17),
with at least one biaxial joint (22), via which the handle part (12) and the tool part (11) are connected together and which has a first joint axis (23) and a second joint axis (24), wherein the first joint axis (23) is arranged at a right angle to the longitudinal axis (24) and the second joint axis (24) is arranged transversely to the handle part (12),
**characterised in that** the first joint axis (23) is arranged lying in the handle part (12).

2. Instrument according to claim 1, **characterised in that** the activatable tool element (17) is arranged movably and is connected to the actuating element (26) via a gear mechanism (34).

3. Instrument according to claim 1 or 2, **characterised in that** both the first and the second joint axes (23, 24) each have a rotational range limitation (50).

4. Instrument according to claim 1, **characterised in that** the joint (22) is a ball joint with a joint ball (30) and a joint socket (29).

5. Instrument according to claim 1, **characterised in that** the joint (22) has a catch device (56) for fixing set relative positions of the handle part (12) and tool part (11).

6. Instrument according to claim 4 and 5, **characterised in that** the catch device (56) is formed by at least one protrusion (58) and depressions (57) which are arranged on the joint ball (30) and the joint socket (29).

7. Instrument according to claim 4, **characterised in that** joint ball (30) is connected to the handle part (12) and the joint socket (29) is connected to the tool part (11).

8. Instrument according to claim 4, **characterised in that** the joint ball (30) has a passage channel (31) which connects an interior of the tool part (11) to an interior of the handle part (12).

9. Instrument according to claim 2 and 4, **characterised in that** a force transmission element (32) belonging to the gear mechanism (34) is arranged in the passage channel (31).

10. Instrument according to claim 2, **characterised in that** the gear mechanism (34) is a lever linkage.

11. Instrument according to claim 9, **characterised in that** the force transmission element (32) comprises a flexible portion (42) serving as a joint (38).

12. Instrument according to claim 11, **characterised in that** the portion (43) is a film hinge (44).

13. Instrument according to claim 9, **characterised in that** the force transmission element (32) is a flexible traction means (45).

14. Instrument according to claim 1, **characterised in that** the handle part (12) comprises a switch (61) to allow activation of the tool (16).

## Revendications

1. Instrument chirurgical (10),
comprenant une partie à outil (11) qui présente une tige (14) disposée le long d'un axe longitudinal (13) et dotée d'un outil (16) disposé à son extrémité distale (15) et comportant au moins un élément d'outil (17) susceptible d'être activé,
comprenant une partie de préhension (12) qui présente un manche (25) et un élément d'actionnement (26) qui est en liaison fonctionnelle avec l'élément d'outil (17) susceptible d'être activé,
comprenant une articulation (22) dotée d'au moins deux axes, par l'intermédiaire de laquelle la partie de préhension (12) et la partie à outil (11) sont reliées l'une à l'autre, et qui présente un premier axe d'articulation (23) et un deuxième axe d'articulation (24), le premier axe d'articulation (23) étant disposé à angle droit par rapport à l'axe longitudinal (13) et le deuxième axe d'articulation (24) étant disposé transversalement à la partie de préhension (12),
**caractérisé en ce que**
le premier axe d'articulation (23) est disposé de manière à se situer dans la partie de préhension (12).

2. Instrument selon la revendication 1, **caractérisé en ce que** l'élément d'outil (17) susceptible d'être activé est monté de façon mobile et est relié à l'élément d'actionnement (26) par l'intermédiaire d'un mécanisme de transmission (34).

3. Instrument selon la revendication 1 ou 2, **caractérisé en ce que** aussi bien le premier que le deuxième axe d'articulation (23, 24) présentent respectivement un moyen de limitation de plage de rotation (50).

4. Instrument selon la revendication 1, **caractérisé en ce que** l'articulation (22) est une articulation sphérique comprenant une rotule d'articulation (30) et un coussinet à rotule (29).

5. Instrument selon la revendication 1, **caractérisé en ce que** l'articulation (22) présente un dispositif d'encliquetage (56) destiné à fixer des positions relatives réglées de la partie de préhension (12) et de la partie à outil (11).

6. Instrument selon la revendication 4 et 5, **caractérisé en ce que** le dispositif d'encliquetage (56) est constitué d'au moins une saillie (58) et de creux (57) qui sont disposés sur la rotule d'articulation (30) et le coussinet à rotule (29).

7. Instrument selon la revendication 4, **caractérisé en ce que** la rotule d'articulation (30) est reliée à la partie de préhension (12), et **en ce que** le coussinet à rotule (29) est relié à la partie à outil (11).

8. Instrument selon la revendication 4, **caractérisé en ce que** la rotule d'articulation (30) présente un conduit de passage (31) qui relie un espace interne de la partie à outil (11) à un espace interne de la partie de préhension (12).

9. Instrument selon les revendications 2 et 4, **caractérisé en ce qu'**il est prévu dans le conduit de passage (31), un élément de transmission de force (32) qui fait partie du mécanisme de transmission (34).

10. Instrument selon la revendication 2, **caractérisé en ce que** le mécanisme de transmission (34) est une tringlerie à levier.

11. Instrument selon la revendication 9, **caractérisé en ce que** l'élément de transmission de force (32) présente une partie souple (43) servant d'articulation (38).

12. Instrument selon la revendication 11, **caractérisé en ce que** la partie (43) est une charnière-film (44).

13. Instrument selon la revendication 9, **caractérisé en ce que** l'élément de transmission de force (32) est un moyen de traction (45) souple.

14. Instrument selon la revendication 1, **caractérisé en ce que** la partie de préhension (12) comprend un interrupteur (61) destiné à permettre l'activation de l'outil (16).
